# EUROPÄISCHE PATENTSCHRIFT

(11) **EP 0 535 506 B1**
(45) Veröffentlichungstag und Bekanntmachung des Hinweises auf die Patenterteilung: **17.04.1996**
(21) Anmeldenummer: 92116200.4
(22) Anmeldetag: 22.09.1992
(51) Int. Cl.: A61B 17/00, A61B 17/28

(54) **Instrument zum Applizieren einer Adhäsionsprophylaxe für endoskopische Eingriffe**
Instrument for applying an adhesion prophylaxis, especially a foil for endoscopic operations
Instrument pour appliquer une prophylaxie d'adhésion, notamment une feuille, pour interventions endoscopiques

(30) Priorität: 02.10.1991 DE 4132855
(43) Veröffentlichungstag der Anmeldung: 07.04.1993
(73) Patentinhaber: WISAP GESELLSCHAFT FÜR WISSENSCHAFTLICHEN APPARATEBAU MBH, D-82054 Sauerlach (DE)
(72) Erfinder: Semm, Kurt, o.Prof.Dr.med.Dr.med.vet.h.c., W-2300 Kiel (DE)
(74) Vertreter: Heim, Hans-Karl, Dipl.-Ing.

(56) Entgegenhaltungen:
- WO-A-92/06638
- US-A- 4 769 038
- US-A- 5 007 895
- US-A- 5 037 379

## Beschreibung

Die Erfindung betrifft ein Instrument zum Applizieren einer Adhäsionsprophylaxe für endoskopische Eingriffe.

In der herkömmlichen Chirurgie ist es bekannt, insbesondere im Abdominalbereich eine Folie in das Abdomen einzusetzen, die die Bildung von Verwachsungen zwischen inneren Organen und der Bauchdecke verhindern soll.

In der US-A-5,007,895 ist ein Instrument beschrieben, welches zur Wundbehandlung mit einem Gazestreifen vorgesehen ist. Der Gazestreifen wird am proximalen Ende des Instrumentes an einer Schneide mit einer erforderlichen Länge abgetrennt, in eine Nut an einem dickeren distalen Ende des Instrumentes eingeklemmt und in die Wunde eingeführt. Die Nut ist derart ausgebildet, daß die Gaze beim Zurückziehen des Instrumentes in der Wunde verbleibt. Das Instrument weist einen vorzugsweise flexiblen, hohlen Schaft auf, um eine antiseptische Aspiration der Wunde durchführen zu können. Die erwähnte Druckschrift offenbart nicht den Einsatz des Instruments für endoskopische Eingriffe. Ferner ist das Instrument nicht derart ausgebildet, daß der Gasestreifen kraft- und/oder formschlüssig innerhalb der vorgesehenen Hülse aufgenommen werden kann.

Die Verwendung von Adhäsionsprophylaxen, insbesondere in Form von Folien, wäre ebenfalls für die endoskopische Chirurgie wünschenswert. Sie scheiterte jedoch bisher an der Forderung, daß die Folien zum einen möglichst wenig geknittert werden und zum anderen auf möglichst einfachem Weg auf ihre volle Größe entfaltbar sein sollten.

Es ist daher die **Aufgabe** der Erfindung, ein Instrument der oben genannten Art zu schaffen, das die Applikation einer folienartigen Adhäsionsprophylaxe auch für endoskopische Eingriffe ermöglicht.

Diese Aufgabe wird erfindungsgemäß durch die Merkmale des Anspruchs 1 gelöst. Vorteilhafte Weiterbildungen der Erfindung sind Gegenstand der Unteransprüche.

Das Instrument zum Applizieren einer Adhäsionsprophylaxe mit einem vorderen Aufnahmebereich für die Adhäsionsprophylaxe weist erfindungsgemäß einen stabförmigen Folienträger auf, der mit einem Längsschlitz in dem vorderen Aufnahmebereich zur kraft- und/oder formschlüssigen Aufnahme einer folienartigen Adhäsionsprophylaxe versehen ist. Der stabförmige Folienträger ist wenigstens im Bereich des Längsschlitzes mit der folienartigen Adhäsionsprophylaxe in einer kreiszylinderförmigen Applikationshülse aufgenommen und wird entweder direkt oder indirekt über eine Trokarhülse in ein Pneumoperitoneum eingeführt.

Im einfachsten Fall kann der vordere Aufnahmebereich mit einem axialer Langsschlitz versehen sein. Dieser Längsschlitz hat vorzugsweise eine Länge von ungefähr 20 cm. Die Folie wird ungefähr mittig in den Längsschlitz eingeführt und anschließend durch Drehen des Folienträgers auf diesem aufgerollt. Der Folienträger mit der aufgerollten Folie wird dann in die Applikationshülse eingeführt, die wiederum über eine Trokarhülse oder direkt in die Bauchdecke eingeführt werden kann. Vorzugsweise hat der geschlitzte Bereich des Folienträgers einen Durchmesser von etwa 5 mm, während die Applikationshülse einen Außendurchmesser von etwa 10 mm aufweisen kann. Die aufgewickelte Folie ist dann in dem Zwischenraum zwischen Folienträger und Applikationshülse angeordnet. An dem geschlitzten Bereich des Folienträgers von 20 cm Länge schließt sich ein ungeschlitzter Bereich von etwa 30 cm Länge an. Der geschlitzte Bereich des Folienträgers kann somit aus der etwa 20 cm langen Applikationshülse vollständig in das Abdomen geschoben und dort durch Aufrollen entfaltet werden. Das Entfalten der Folie kann durch Greifer unterstützt werden, die durch weitere Trokarhülsen in das Pneumoperitoneum eingeführt werden.

Bei der Ausbildung des Aufnahmebereichs des Folienträgers als Längsschlitz wird die Folie oder ein aufblasbares Folienkissen in etwa mittig in den Längsschlitz eingeführt, so daß die Folie zu beiden Seiten etwa gleich weit aus dem Schlitz heraushängt.

Der Aufnahmebereich des Folienträgers kann jedoch auch als Klemmvorrichtung zur Aufnahme eines Längsendes der Folie ausgebildet sein. In dem Aufnahmebereich sollten dann zumindest zwei Anlageflächen ausgebildet sein, die gegeneinander andrückbar sind, so daß das Längsende der Folie festgehalten wird. Ein Lösen der Folie erfolgt nach dem Abrollen im Abdomen lediglich durch Lösen der Anlageflächen.

Die aufgerollte Folie wird vorzugsweise an ihren Ecken in den entsprechenden Bereichen des Abdomens fixiert, so daß eine Verschiebung der Lage der Folie ausgeschlossen ist. Bei Verwendung einer kissenartig aufblasbaren Folie für eine gewünschte stärkere Beabstandung der inneren Organe von der Bauchdecke kann die Fixierung einfach erfolgen, da die Formstabilität des Folienkissens per se gegeben ist.

Die Folie bzw. das Kissen kann z.B. nach etwa zwei bis drei Tagen auf endoskopische Weise wieder entfernt werden. Die Entfernung der Folie kann wiederum durch das erfindungsgemäße Instrument erfolgen.

Das Instrument ermöglicht ein endoskopisches Einbringen der Folie unter Erzielung wesentlicher Erleichterungen verglichen mit der bisherigen operativen Chirurgie. Dort wird die Folie unter Vollnarkose und im Sectiobereich durch eine Art Reißverschluß eingebracht, wobei fast täglich ein Austausch der Folie bzw. eine Beurteilung des Heilungsprozesses im Bereich der Folie durchgeführt wird.

Der Folienträger ermöglicht die Aufnahme sowohl einfacher, steriler dünner Folien als auch von mehrlagigen Folien, die zu Luftkissen aufblasbar sind.

Die Verwendung der Applikationshülse oder einfach Applikator genannt, ermöglicht eine leichtere Drehung des Folienträgers zum Auf- und Abwickeln der Folie. Diese Drehung könnte bei direktem Einsetzen des Folienträgers in eine Trokarhülse durch das Ventil der Trokarhülse behindert werden. Bei Verwendung des Applikators wird das Ventil durch die Aussenwand des Applikators automatisch in Öffnungsstellung gehalten, so daß der Folienträger ohne Behinderung gedreht werden kann.

Die Erfindung wird nachfolgend beispielsweise anhand der schematischen Zeichnung beschrieben. In dieser zeigen:
- Fig. 1: eine perspektivische Ansicht einer Applikationshülse bzw. eines Applikators;
- Fig. 2: eine perspektivische Ansicht des stabförmigen Folienträgers zum Einsatz in den Applikator aus Fig. 1 und
- Fig. 3a bis 3c: die Frontansicht auf den Folienträger am Beginn und am Ende des Aufwickelvorgangs der Folie mit am Ende aufgeschobener Applikationshülse.

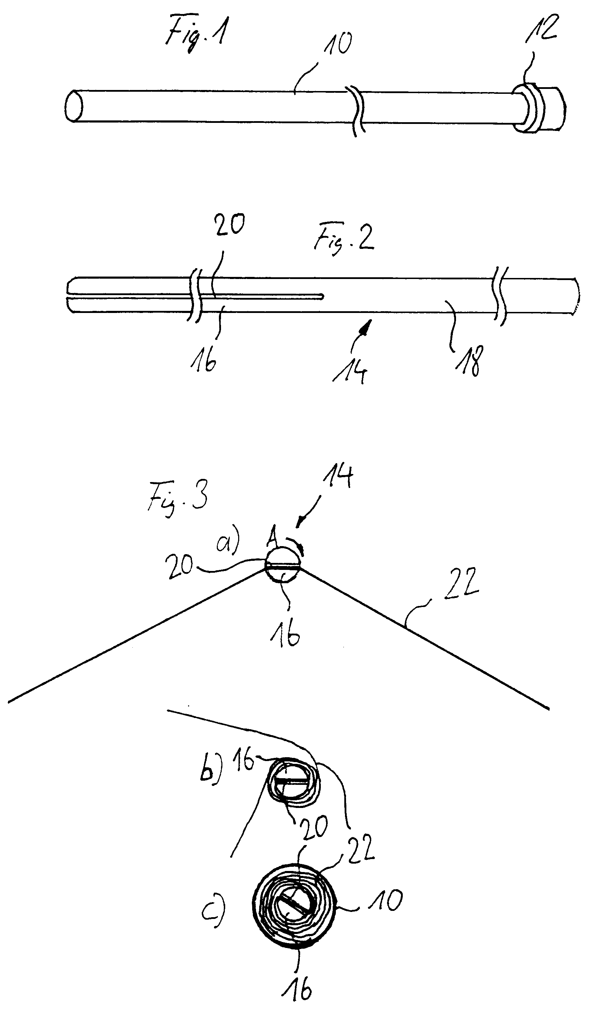

Fig. 1 zeigt eine Applikationshülse 10 mit einem Anschlagring 12 zur Anlage an den Hauptkörper einer Trokarhülse. Die Applikationshülse 10 dient zur Aufnahme eines in Fig. 2 dargestellten Folienträgers 14. Der Folienträger ist unterteilt in einen längsgeschlitzen vorderen Bereich 16 von etwa 20 cm Länge und einen hinteren, ungeschlitzten Bereich 18 von ungefähr 30 cm Länge. Der Applikator 10 hat eine Länge von etwa 20 cm. Der Längsschlitz 20 von z.B. 1 bis 1,5 mm Schlitzbreite im vorderen Bereich 16 des Folienträgers 14 dient als Aufnahmebereich für eine Folie 22 (Fig. 3).

Die Folie wird - wie in Fig. 3 dargestellt - in den Längsschlitz 20 des Folienträgers 14 eingefädelt und anschliessend durch Drehung des Folienträgers in Richtung A auf diesen aufgerollt. Da der Folienträger 14 einen Außendurchmesser von etwa 5 mm hat und der dünnwandige Applikator 10 einen Außendurchmesser von etwa 10 mm, wird die aufgerollte Folie 22 wie in Fig. 3c dargestellt, in dem verbleibenden Freiraum untergebracht. Die Länge des Applikators ist dabei so bemessen, daß der vordere geschlitzte Bereich 16 des Folienträgers 14 in seiner gesamten Länge in dem Applikator 10 aufgenommen werden kann. Der Applikator 10 ist mit seinem Außendurchmesser von etwa 10 mm wiederum für den Einsatz in eine Trokarhülse geeignet. Durch den Applikator 10 ist der Folienträger drehbar gehalten, wobei die Drehung nicht durch das Ventil der Trokarhülse behindert wird. Der Applikator 10 wird soweit auf die Trokarhülse aufgeschoben, bis der Anlagering 12 an den Hauptkörper der Trokarhülse anliegt. Der Applikator kann an seinem rückwärtigen Ende eine Dichtung zum Folienträger 14 aufweisen, wodurch ein Austreten von Gas zwischen dem Applikator 10 und dem hinteren Ende 18 des Folienträgers 14 vermieden wird. Ein Entweichen von Luft läßt sich jedoch auch dann weitgehend verhindern, wenn der Außendurchmesser des hinteren Bereichs 18 des Folienträgers nahezu dem Innendurchmesser des Applikators 10 entspricht. Durch die axiale Bewegung des Folienträgers 14 in Richtung auf das Abdomen wird der vordere Bereich 16 des Folienträgers 14 aus dem Applikator 10 herausgeschoben. Durch anschließendes Drehen des Folienträgers 14 wird die aufgewickelte Folie 22 im Abdomen entfaltet, wobei die Entfaltung durch weitere endoskopische Instrumente, wie z.B. Greifer unterstützt werden kann, die durch andere Trokarhülsen in das Pneumoperitoneum eingeführt werden.

Die Folien können sowohl aus biologisch resistentem Material als auch aus einem Material bestehen, das sich im Laufe der Zeit auflöst. Die Folie verhindert Verwachsungen zwischen der Bauchdecke und den an der Bauchdecke anliegenden inneren Organen in der Zeit nach dem operativen Eingriff. Die Folie kann nach etwa zwei bis drei Tagen durch Einführen des Folienträgers und Aufwickeln der Folie wieder entfernt werden. Bei Verwendung einer sich selbst auflösenden Folie erübrigt sich ein weiterer endoskopischer Eingriff.

Das erfindungsgemäße Instrument eröffnet daher als endoskopischer Folienapplikator eine elegante Adhäsionsprophylaxe bei endoskopischen Eingriffen.

## Patentansprüche

1. Instrument zum Applizieren einer Adhäsionsprophylaxe für endoskopische Eingriffe mit einem stabförmigen Folienträger (10), der einen vorderen, als Längsschlitz (20) ausgebildeten Aufnahmebereich (16) für die Aufnahme einer folienartigen Adhäsionsprophylaxe (22) aufweist, und mit einer den
stabförmigen Folienträger (14) wenigstens im Bereich des Längsschlitzes (20) aufnehmenden Kreiszylinderförmigen Applikationshülse (10), die in einer Trokarhülse oder direkt in ein Pneumoperitoneum einführbar ist, wobei die Adhäsionsprophylaxe (22) innerhalb der Applikationshülse (10) kraft- und/oder formschlüssig aufgenommen wird.

2. Instrument nach Anspruch 1,
dadurch **gekennzeichnet**,
daß der Längsschlitz (20) des Aufnahmebereiches (16) als ein axial verlaufender Längsschlitz (20) ausgebildet ist und daß die folienartige Adhäsionsprophylaxe (22) in den Längsschlitz (20) mittig einführbar ist und durch Drehen des Folienträgers (14) auf diesen aufrollbar ist.

3. Instrument nach Anspruch 1 oder 2,
dadurch **gekennzeichnet**,
daß der vordere Aufnahmebereich (16) des Folienträgers (14) mit dem Längsschlitz (20) in seiner gesamten Länge in der Applikationshülse (10) aufgenommen ist.

4. Instrument nach einem der Ansprüche 1 bis 3,
dadurch **gekennzeichnet**,
daß der Folienträger (14) als ein Rundstab mit einer Gesamtlänge, die größer als die zweifache Länge des Längsschlitzes (20) ist, ausgebildet ist.

5. Instrument nach einem der vorhergehenden Ansprüche,
dadurch **gekennzeichnet**,
daß das Längsende des Folienträgers (14) abgerundet ist.

6. Instrument nach einem der vorhergehenden Ansprüche,
dadurch **gekennzeichnet**,
daß eine Dichtung zwischen der Applikationshülse (10) und dem Folienträger (14) vorgesehen ist.

7. Instrument nach einem der vorhergehenden Ansprüche,
dadurch **gekennzeichnet**,
daß der Folienträger (14) neben dem vorderen Aufnahmebereich (16) mit dem Längsschlitz (20) einen hinteren Bereich (18) aufweist, daß der vordere Aufnahmebereich (16) einen kleineren Außendurchmesser als der hintere Bereich (18) aufweist und daß der Außendurchmesser des hinteren Bereichs (18) des Folienträgers (14) dem Innendurchmesser der Applikationshülse (10) entspricht.

8. Instrument nach einem der vorhergehenden Ansprüche,
dadurch **gekennzeichnet**,
daß der Außendurchmesser der Applikationshülse (10) etwa dem Innendurchmesser einer Trokarhülse entspricht.

9. Instrument nach einem der vorhergehenden Ansprüche,
dadurch **gekennzeichnet**,
daß die Applikationshülse (10) einen radial umlaufenden Anschlagbereich (12) für die Anlage an dem Hauptkörper der Trokarhülse aufweist.

10. Instrument nach einem der vorhergehenden Ansprüche,
dadurch **gekennzeichnet**,
daß der Aufnahmebereich nutenförmig ausgebildet ist.

11. Instrument nach einem der vorhergehenden Ansprüche,
dadurch **gekennzeichnet**,
daß im Aufnahmebereich gegeneinander verstellbare Anlageflächen ausgebildet sind.

12. Instrument nach Anspruch 11,
dadurch **gekennzeichnet**,
daß die Anlageflächen aufgerauht, geriffelt oder strukturiert ausgebildet sind.

## Claims

1. An instrument for application of an adhesion prophylaxis for endoscopic surgery, comprising a rod-shaped film carrier (14), which has a front reception area (16) formed as an elongated slot (20) for the reception of a film-like adhesion prophylaxis (22), and a circular cylindrical application sleeve (10), receiving the rod-shaped film carrier (14) at least in the area of the elongated slot (20), whereby the circular cylindrical application sleeve (10) can be inserted into a trocar cannula or directly into a pneumoperitoneum and the adhesion prophylaxis (22) is received within the application sleeve (10) in an frictional and/or positive locking manner.

2. An instrument according to claim 1,
**characterized** in that
the elongated slot (20) of the reception area (16) is formed as an axially running elongated slot (20) and that the film-like adhesion prophylaxis (22) can be centrally inserted into the elongated slot (20) and wound up onto the film carrier (14) by rotation of the latter.

3. An instrument according to claim 1 or 2,
**characterized** in that
the application sleeve (10) receives the front reception area (16) of the film carrier (14) with the elongated slot (20) in its entire length.

4. An instrument according to one of the claims 1 to 3,
**characterized** in that
the film carrier (14) is constructed as a round rod with a total length, which is greater than twice the length of the elongated slot (20).

5. An instrument according to one of the preceding claims,
**characterized** in that
the longitudinal end of the film carrier (14) is rounded.

6. An instrument according to one of the preceding claims,
**characterized** in that
a seal is provided between the application sleeve (10) and the film carrier (14).

7. An instrument according to one of the preceding claims,
**characterized** in that
the film carrier (14) has in addition to the front reception area (16) with the elongated slot (20) a rear area (18), that the front reception area (16) has a smaller external diameter than the rear area (18) and that the external diameter of the rear area (18) of the film carrier (14) corresponds to the internal diameter of the application sleeve (10).

8. An instrument according to one of the preceding claims,
**characterized** in that
the external diameter of the application sleeve (10) substantially corresponds to the internal diameter of a trocar cannula.

9. An instrument according to one of the preceding claims,
**characterized** in that
the application sleeve (10) has a radially, round-running stopping area (12) for the engagement to the main body of the trocar cannula.

10. An instrument according to one of the preceding claims,
**characterized** in that
the reception area is formed as a slot.

11. An instrument according to one of the preceding claims,
**characterized** in that
bearing surfaces, adjustable against one another, are formed in the reception area.

12. An instrument according to claim 11,
**characterized** in that
the bearing surfaces are roughened, grooved or structured surfaces

## Revendications

1. Instrument pour appliquer une prophylaxie des adhérences pour des interventions endoscopiques, avec un support de feuille (14) en forme de tige, qui présente une zone de réception avant (16) en forme de fente longitudinale (20) destinée à recevoir une prophylaxie des adhérences (22) de type feuille, et avec une douille d'application (10) de forme cylindrique circulaire recevant le support de feuille (14) en forme de tige au moins au niveau de la fente longitudinale (20), douille qui peut être introduite dans une douille de trocart ou directement dans un pneumopéritoine, la prophylaxie d'adhésion (22) étant logée à l'intérieur de la douille d'application (10) en engagement positif et/ou en ajustement serré.

2. Instrument selon la revendication 1,
**caractérisé** en ce que la fente longitudinale (20) de la zone de réception (16) est formée en fente longitudinale (20) orientée dans le sens axial, et en ce que la prophylaxie d'adhésion (22) de type feuille peut être introduite de manière centrale dans la fente longitudinale (20) et peut être enroulée sur le support de feuille (14) par rotation de celui-ci.

3. Instrument selon la revendication 1 ou 2,
**caractérisé** en ce que la zone de réception avant (16) du support de feuille (14) comportant la fente longitudinale (20) se loge sur toute sa longueur dans la douille d'application (10).

4. Instrument selon l'une des revendications 1 à 3,
**caractérisé** en ce que le support de feuille (14) est formé en tige ronde d'une longueur totale qui est supérieure au double de la longueur de la fente longitudinale (20).

5. Instrument selon l'une des revendications précédentes,
**caractérisé** en ce que l'extrémité longitudinale du support de feuille (14) est arrondie.

6. Instrument selon l'une des revendications précédentes,
**caractérisé** en ce qu'un joint est prévu entre la douille d'application (10) et le support de feuille (14).

7. Instrument selon l'une des revendications précédentes,
**caractérisé** en ce que le support de feuille (14), outre la zone de réception avant (16) avec la fente longitudinale (20), présente également une zone arrière (18),
en ce que la zone de réception avant (16) présente un diamètre extérieur plus petit que celui de la zone arrière (18), et
en ce que le diamètre extérieur de la zone arrière (18) du support de feuille (14) correspond au diamètre intérieur de la douille d'application (10).

8. Instrument selon l'une des revendications précédentes,
**caractérisé** en ce que le diamètre extérieur de la douille d'application (10) correspond approximativement au diamètre intérieur d'une douille de trocart.

9. Instrument selon l'une des revendications précédentes,
**caractérisé** en ce que la douille d'application (10) présente une zone de butée (12) l'entourant dans le sens radial et destinée à s'appuyer contre le corps principal de la douille de trocart.

10. Instrument selon l'une des revendications précédentes,
**caractérisé** en ce que la zone de réception est formée en rainure.

11. Instrument selon l'une des revendications précédentes,
**caractérisé** en ce que des surfaces d'appui mobiles l'une par rapport à l'autre sont formées dans la zone de réception.

12. Instrument selon la revendication 11,
**caractérisé** en ce que les surfaces d'appui sont formées rugueuses, moletées ou structurées.
